# EUROPEAN PATENT APPLICATION

(11) **EP 3 646 892 A1**
(43) Date of publication of application: **06.05.2020**
(21) Application number: 18203584.0
(22) Date of filing: 31.10.2018
(51) Int. Cl.: A61L 2/18

(54) **DENTAL OR MEDICAL DEVICE FOR SANITIZING OR MAINTENANCE OF A LOAD**

(71) Applicant: W & H Sterilization S.r.l., 24060 Brusaporto (BG) (IT)
(72) Inventor: MAGNO, Luigi Marino, 24031 Almenno San Salvatore (IT); EIBL, Johann, 5230 Mattighofen (AT)
(74) Representative: Benda, Ralf

(57) **Abstract**

A dental or medical device (1) for sanitizing or maintenance of a load having an outer housing (2), a chamber (3) at least partially arranged in the outer housing (2) and configured to accommodate the load to be sanitized or maintained, at least one supply device (4) for supply of at least one sanitizing or maintaining medium into the chamber (3) and a light emission device (5) which emits visible light into the interior of the chamber (3) or is arranged on an inner side (7A) of a door (7) which closes a loading opening (6) of the chamber (3) or is arranged on a wall (8) surrounding the loading opening (6) of the chamber (3) to indicate the sanitizing or maintenance status of the load accommodated in the chamber (3).

## Description

The present invention relates to a dental or medical device for sanitizing or maintenance of a load, in particular at least one dental or medical instrument, which is configured to indicate the sanitizing or maintenance status of the load.

A dental or medical device for sanitizing or maintenance of a load in form of a sterilizer is known, for example, from patent US 9,522,044 B2. A display is provided on the outer housing of the sterilizer which displays a plurality of values of parameters in connection with the operation or status of the sterilizer.

The large number of parameters shown on the display is disadvantageous and confusing for a user. There is the danger that a user may oversee, mix up or misinterpret values.

It is an object of the present application to overcome these deficiencies and to provide in particular a dental or medical device for sanitizing or maintenance with an improved indication of values of parameters which is clearer and easier to discern for a user.

These objects are achieved by a dental or medical device for sanitizing or maintenance of a load, in particular at least one dental or medical instrument, according to Claims 1 and 2. Particularly advantageous embodiments are defined in the dependent claims.

The dental or medical device for sanitizing or maintenance of a load, in particular at least one dental or medical instrument, according to the invention comprises an outer housing, a chamber at least partially arranged in the outer housing and configured to accommodate the load to be sanitized or maintained, and at least one supply device for supply of at least one sanitizing or maintaining medium into the chamber to sanitize or maintain the load.

According to a first embodiment the dental or medical device for sanitizing or maintenance of a load further comprises a light emission device which is configured (and positioned) to emit visible light (visible for the eye of a human being) into the interior of the chamber to indicate the sanitizing or maintenance status of the load accommodated in the chamber.

According to a second, alternative embodiment the dental or medical device for sanitizing or maintenance of a load further comprises a light emission device which is configured to emit visible light (visible for the eye of a human being) to indicate the sanitizing or maintenance status of the load accommodated in the chamber and which is arranged on an inner side of a door which closes or covers a loading opening of the chamber or on a wall surrounding and/ or adjoining the loading opening of the chamber.

Preferably, the wall surrounding the loading opening of the chamber is covered by the door when the door (and the loading opening) is closed. Preferably, the wall surrounding the loading opening of the chamber connects to the outer housing. Preferably, the inner side of the door is the side which faces the loading opening of the chamber.

According to the invention, the indication of the sanitizing or maintenance status of the load is no longer shown on the display on the front side of the outer housing but through the light emission device which emits visible light into the interior of the chamber or from an inner side of the door which closes the loading opening of the chamber or from a wall surrounding and/ or adjoining the loading opening. Thus, the sanitizing or maintenance status of the load is easily and clearly discernable for a user and the danger that the user mixes up or misinterprets values is greatly reduced. In addition, the user advantageously sees the sanitizing or maintenance status of the load when he/ she handles it, for example, takes it out of the chamber.

The light emission device emitting visible light into the interior of the chamber to indicate the sanitizing or maintenance status of the load can in addition be used to illuminate the interior of the chamber, in particular to improve the visibility for the user in the chamber. Alternatively, the light emission device emitting visible light into the interior of the chamber to indicate the sanitizing or maintenance status of the load is a first light source, whereas a second light source, in particular a light source emitting white light, is provided to illuminate the chamber, so that the first light emission device is mainly provided to indicate the sanitizing or maintenance status of the load.

The dental or medical device for sanitizing or maintenance may comprise various devices, for example, at least one of: a sterilizer, in particular a steam sterilizer; a disinfector, in particular a thermodisinfector; a chemiclave; a maintenance or care device for supply of a lubricant and/ or any other maintenance medium to a medical or dental instrument.

The chamber of the dental or medical device for sanitizing or maintenance is preferably made of metal or plastic. Preferably, the chamber has a cylindrical shape. The chamber comprises a loading opening to place the load into the chamber and take the load out of the chamber.

Preferably, the chamber comprises a second opening through which at least a portion of the visible light generated and/ or emitted by the light emission device and/ or at least a part of the light emission device enters the chamber, in particular a light source which generates the visible light, an optical light guide or an electric line for supplying electric energy to the light source. Preferably, the light source, the optical light guide or the electric line extends through the second opening into the interior of the chamber. Preferably the light source which generates the visible light is arranged in the second opening of the chamber and/ or mounted adjacent to the second opening to emit at least a portion of the visible light into the chamber.

Preferably, the loading opening and the second opening are formed by different and/ or separated openings. Preferably the loading opening and the second opening are arranged on different sides or surfaces of the chamber. Preferably, the loading opening and the second opening are arranged on opposite sides of the chamber, for example, on a top wall and a bottom wall of the chamber. Alternatively, the loading opening and the second opening are arranged on adjacent side walls, for example, on a top wall and on a lateral wall or side wall of the chamber. The provision of different and/ or separated openings makes it possible in an advantageous manner to position the second opening such that the visible light or a light source of the light emission device can be easily and optimally seen by the user.

Alternatively, the loading opening forms the second opening, so that in particular the visible light emitted by the light emission device and/ or at least a part of the light emission device enters the chamber through the loading opening. Accordingly, in an advantageous manner only one opening is necessary for the load and for the visible light or the part of the light emission device. It is in particular advantageous that the loading opening forms the second opening when the light emission device is arranged on an inner side of the door which closes the loading opening or on the wall surrounding the loading opening of the chamber.

When the light emission device is arranged on an inner side of the door or on the wall surrounding the loading opening of the chamber preferably at least a portion of the visible light does not enter the chamber, in particular when the door is not closed. Particularly preferably at least a portion of the visible light is radiated away from the door, the wall and/ or the dental or medical device for sanitizing or maintenance.

Preferably, the light source of the light emission device which generates the visible light is arranged on the inner side of the door of the dental or medical device for sanitizing or maintenance which is configured to close the chamber. Advantageously, this provides good perceptibility of the visible light for the user and there is no need for an optical light guide.

The at least one supply device for supply of at least one sanitizing or maintaining medium into the chamber to sanitize or maintain the load preferably comprises one of the following components: a heater; a vaporizer; a pump; a valve, e.g. a check valve or a throttle valve; a tubing for conveying a sanitizing or maintaining medium; a container for storing a sanitizing or maintaining medium; a connector to couple an instrument to be sanitized or maintained to a tubing for conveying a sanitizing or maintaining medium.

The light emission device preferably has a light source which generates the visible light. The light source preferably comprises at least one of: an optical semiconductor element, in particular an LED; an incandescent light; a high pressure lamp; a fluorescent lamp.

The light source can be disposed in the dental or medical device for sanitizing or maintenance or outside of the dental or medical device for sanitizing or maintenance. When the light source is arranged in the dental or medical device for sanitizing or maintenance it can be positioned in particular in the chamber, in the second opening or outside of the chamber, for example, in a space between the outer housing and a wall of the chamber. When the light source is arranged in the second opening or in the chamber advantageously the majority of the visible light generated by the light source is emitted into the chamber.

Preferably, an electric line for supplying electric energy to the light source is provided which electrically connects the light source to a power supply. Preferably, the electric line reaches to and/ or extends through the second opening, in particular if the light source is arranged in the chamber or in the second opening.

The light emission device preferably has an optical light guide configured to direct at least a portion of the visible light generated by the light source away from the light source, in particular into the chamber. Preferably, the optical light guide reaches to and/ or extends through the second opening. Preferably, the optical light guide comprises at least one optic fibre. Due to the provision of the optical light guide the light source need not be arranged in the chamber, which makes the manufacture of the light emission device easier and cheaper.

The door which closes the loading opening of the chamber preferably defines a front side or forms at least a section of the front side of the dental or medical device for sanitizing or maintenance. The door is preferably hinged to the dental or medical device for sanitizing or maintenance.

The dental or medical device for sanitizing or maintenance preferably has a controller, in particular a microcontroller, and at least one sensor communicatively coupled with the controller. The sensor is configured to detect a value of at least one operating parameter of the dental or medical device for sanitizing or maintenance and to transmit the detected value to the controller. The controller is configured to receive the detected value, to process it and to control the light emission device based on the detected value of at least one operating parameter. The controller preferably comprises a processor to process the detected value. This configuration advantageously provides a secure and reliable indication of the sanitizing or maintenance status of the load accommodated in the chamber by the light emission device. The controller is preferably arranged in the dental or medical device for sanitizing or maintenance.

The sensor preferably comprises at least one of: a temperature sensor; a humidity sensor; a pressure sensor; a flowmeter; a timekeeper; a level meter; a conductivity meter. The sensor preferably comprises at least one of: a piezo-electric sensor; an inductive sensor; a capacitive sensor; an optical sensor; an active sensor; a passive sensor.

Based on the detected value of at least one operating parameter the controller is configured to at least one of: turn the light emission device on; turn the light emission device off; vary the light intensity of the visible light emitted by the light emission device; vary the wavelength of the visible light emitted by the light emission device; operate the light emission device in different light emitting modes; operate the light emission device in a continuous light emitting mode; operate the light emission device in a discontinuous or flashing light emitting mode. These operating modes of the light emission device provide a reliable indication of the sanitizing or maintenance status of the load accommodated in the chamber for the user.

In particular when the light source is arranged in the interior of the chamber or in the second opening of the chamber or adjacent the second opening of the chamber it is preferably sealed to withstand the harsh conditions in the chamber during sanitizing or maintenance. Preferably, the light source is hermetically sealed by an enclosure. Preferably, the enclosure comprises at least one portion transparent for visible light, for example, made of glass. Preferably, the enclosure comprises at least one metallic portion. Preferably, electric contacts of the light source for electric power supply penetrate the enclosure. Preferably, the electric contacts of the light source connect to the electric line for supplying electric energy mentioned above.

A display for providing information about or with reference to the dental or medical device for sanitizing or maintenance is preferably arranged on the outer housing, in particular on the front side and/ or next to the door. In particular, the display is configured to indicate information about the performance and/ or an operating status of the dental or medical device for sanitizing or maintenance (but not of the sanitizing or maintenance status of the load accommodated in the chamber).

Preferably, the display comprises at least two, preferably three, light sources which are configured to emit visible light with different wave lengths and/ or emission modes to indicate the operating status. The at least two light sources preferably comprise optical semiconductor elements, in particular LEDs. Each of the at least two light sources preferably can emit visible light with different wave lengths and in particular comprises a plurality of different LEDs in order to be able to emit visible light with different wave lengths. The at least two light sources preferably are configured to emit at least one of the following colors: red; blue; green; yellow; orange; white. The emission modes comprise at least on of: a continuous light emitting mode; a discontinuous or flashing light emitting mode; a running or chasing light emitting mode in which the at least two light sources light up in sequence.

The at least two light sources preferably are configured to commonly indicate the same information, performance or operating status (at a certain moment). In particular, the plurality of light sources all light up in the same color and emission mode to indicate a certain performance or operating status. This advantageously improves the recognisability for the user.

The information indicated by the at least two light sources comprises at least one of: the dental or medical device for sanitizing or maintenance is turned on or off; a sanitizing or maintenance process is running or finished; a sanitizing or maintenance process is stopped before its regular ending; occurrence of an error; need for servicing; need for refill of at least one sanitizing or maintaining medium; a measured value or range of a parameter of the dental or medical device for sanitizing or maintenance, for example, the temperature or the pressure in the chamber; indication that information, statistics and/ or advices for the user is/ are available.

In particular, the display in addition comprises a touch screen, an LCD screen or a similar screen to display at least one of alphanumeric symbols, control elements for a user to select operation modes and/ or values of operating parameters, and graphs. The screen is preferably configured to display details and/ or additional information about the performance or operating status and/ or statistics and/ or advices. Especially preferred the at least two light sources are configured to indicate that said details and/ or additional information and/ or statistics and/ or advices can be retrieved from the screen. Preferably, the at least two light sources are arranged at a distance from the screen.

A controller is preferably provided to control the display having the at least two, preferably three, light sources and in particular the screen. Especially preferably, the dental or medical device for sanitizing or maintenance comprises a single or shared controller which controls the display as well as the light emission device which is configured to emit visible light to indicate the sanitizing or maintenance status of the load.

According to a further embodiment the dental or medical device for sanitizing or maintenance comprises a tray or rack which is configured to support the load, in particular at least one dental or medical instrument, to be sanitized or maintained. The tray or rack can be placed into the chamber of the dental or medical device for sanitizing or maintenance, wherein at least a portion of the light emission device, in particular the light source of the light emission device which generates the visible light is arranged on the tray or rack. This configuration provides indication of the sanitizing or maintenance status of the load (supported by the tray or rack) even when the tray or rack and the load are outside of the chamber or of the dental or medical device for sanitizing or maintenance.

In particular the light emission device and/ or the light source on the tray or rack are configured to provide an indication of the sanitizing or maintenance status of the load supported by the rack or tray for the user through different operating modes of the light source as specified above. The operating modes preferably comprise one of: turning the light emission device on; turning the light emission device off; varying the light intensity of the visible light emitted by the light emission device; varying the wavelength of the visible light emitted by the light emission device; operating the light emission device in different light emitting modes; operating the light emission device in a continuous light emitting mode; operating the light emission device in a discontinuous or flashing light emitting mode.

Preferably, at least a portion of the light emission device and/ or the light source is mounted on a frame of the tray or rack.

Preferably, a battery provides for electric energy supply of the light source of the light emission device, wherein the battery as well as the light source are mounted on the tray or rack. Alternatively, a wireless energy supply for the light source is provided, for example through inductive energy transfer and/ or via radio transmission, i.e. Wi-Fi or Bluetooth.

Preferably, a first transmitter is arranged on the tray or rack and a second transmitter is connected to a component of the dental or medical device for sanitizing or maintenance, for example, to the chamber or controller, to transmit the energy and/ or control signals between the light source on the tray or rack and the dental or medical device for sanitizing or maintenance. The first and second transmitter comprise a coil or an antenna, for example.

A controller is preferably mounted on the tray or rack and connected to the portion of the light emission device or the light source on the tray or rack to control in particular the operation of the light source as specified above.

According to another embodiment a dental or medical sterilizer for sterilization of a load comprises an outer housing, a chamber at least partially arranged in the outer housing and configured to accommodate the load to be sterilized, at least one supply device for supply of at least one sterilizing medium into the chamber to sterilize the load and a display. The display is arranged on the outer housing and comprises at least two, preferably three, light sources which are configured to emit visible light with different wave lengths to provide information about or with reference to the dental or medical device for sanitizing or maintenance.

The display is configured in particular to indicate information about the performance and/ or an operating status of the dental or medical device for sanitizing or maintenance. Preferably, the display in addition comprises a touch screen, an LCD screen or a similar screen. Any further details about the dental or medical sterilizer for sterilization and in particular about the display are described above.

The invention will be described below with reference to the following drawings:

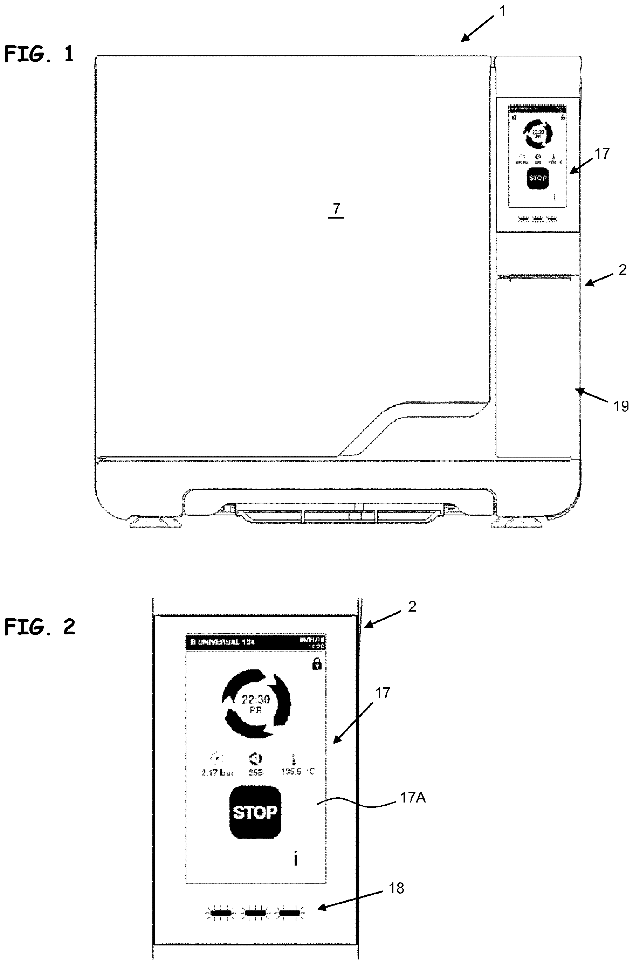

Figure 1 shows a front view of a dental or medical device for sanitizing or maintenance of a load.
Figure 2 shows an enlarged view of the display of the dental or medical device for sanitizing or maintenance of Fig. 1.

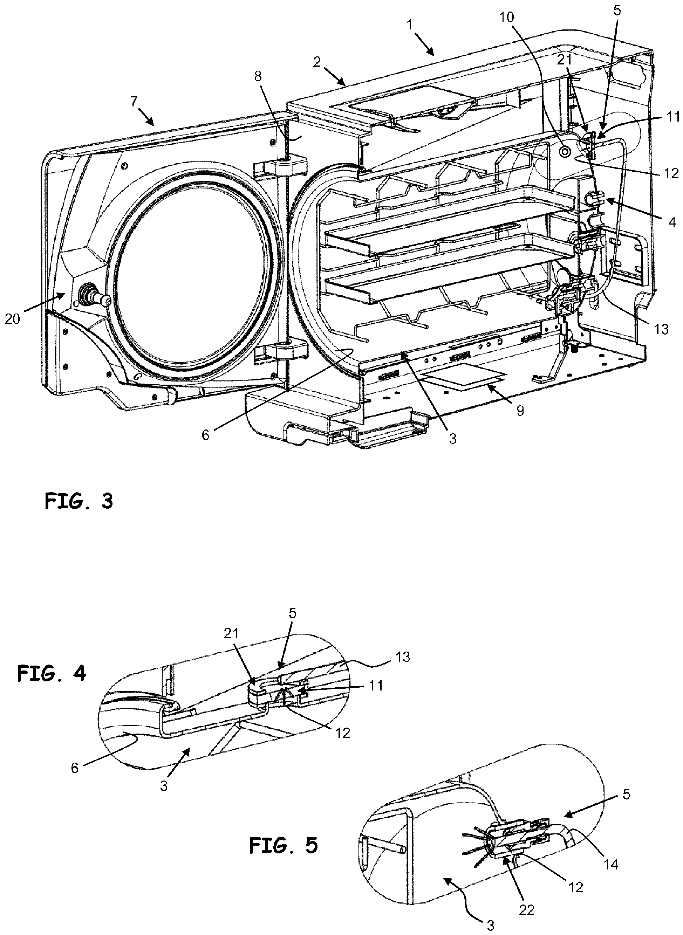

Figure 3 shows a sectional view of a dental or medical device for sanitizing or maintenance of a load designed as a sterilizer and having a first embodiment of a light emission device which is configured to emit visible light into the interior of a sterilizing chamber to indicate the sanitizing or maintenance status of the load accommodated in the sterilizing chamber.
Figure 4 shows a second embodiment of a light emission device which is configured to emit visible light into the interior of a sterilizing chamber to indicate the sanitizing or maintenance status of the load accommodated in the sterilizing chamber.
Figure 5 shows a third embodiment of a light emission device which is configured to emit visible light into the interior of a sterilizing chamber to indicate the sanitizing or maintenance status of the load accommodated in the sterilizing chamber.

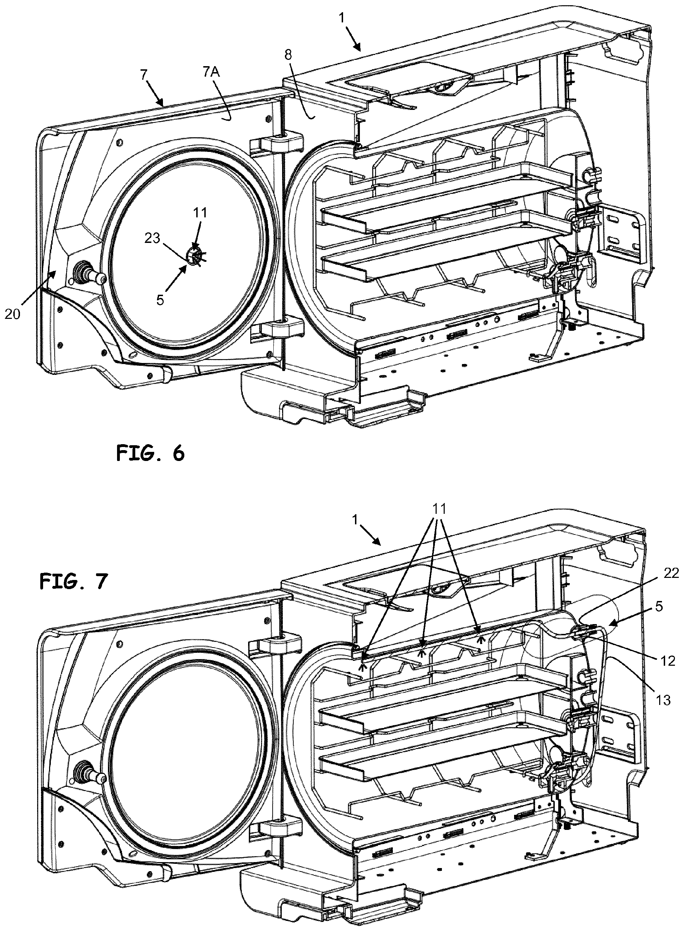

Figure 6 shows a sectional view of a dental or medical device for sanitizing or maintenance of a load designed as a sterilizer and having a light emission device arranged on the door of the sterilizer which is configured to emit visible light to indicate the sanitizing or maintenance status of the load accommodated in the sterilizing chamber.
Figure 7 shows a sectional view of a dental or medical device for sanitizing or maintenance of a load designed as a sterilizer and having a fourth embodiment of a light emission device which is configured to emit visible light into the interior of a sterilizing chamber to indicate the sanitizing or maintenance status of the load accommodated in the sterilizing chamber.

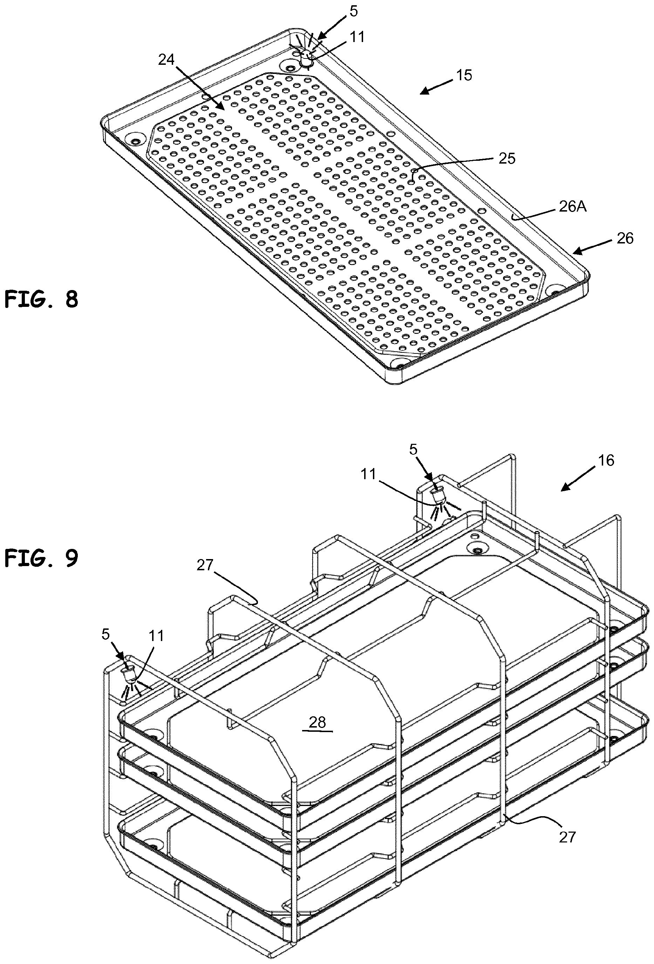

Figure 8 shows a tray which is configured to support the load of a dental or medical device for sanitizing or maintenance and having at least a portion of a light emission device which is configured to emit visible light to indicate the sanitizing or maintenance status of the load supported by the tray.
Figure 9 shows a rack which is configured to support the load of a dental or medical device for sanitizing or maintenance and having at least a portion of a light emission device which is configured to emit visible light to indicate the sanitizing or maintenance status of the load supported by the rack.

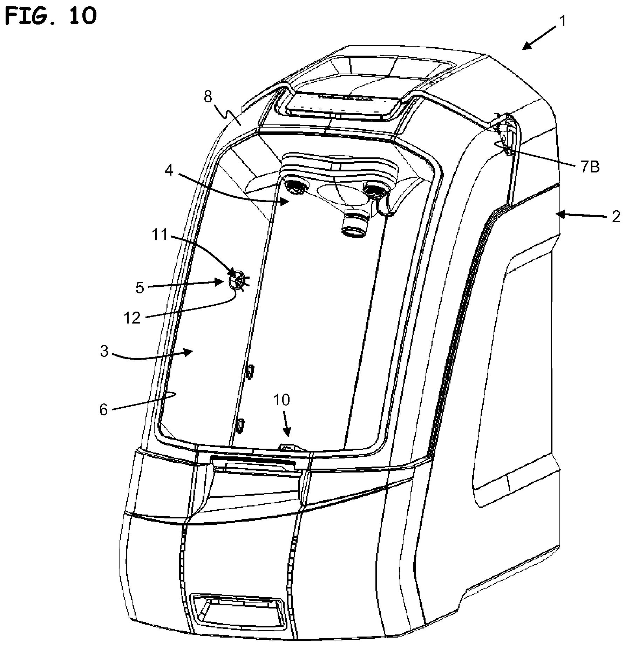

Figure 10 shows an embodiment of a dental or medical device for sanitizing or maintenance of a load designed as a maintaining or care device and having a light emission device which is configured to emit visible light into the interior of a maintenance chamber to indicate the sanitizing or maintenance status of the load accommodated in the maintenance chamber.

Figure 1 shows a front view of a dental or medical device 1 for sanitizing or maintenance of a load. The dental or medical device 1 has an outer housing 2 with a plurality of outer walls including a front side 19. The front side 19 comprises a door 7 which closes the interior of the dental or medical device 1 and provides access to the interior.

On a back side of the dental or medical device 1 opposite to the front side 19 one or more connectors (not shown) are provided to connect the dental or medical device 1 to external sources, in particular to an electric power source and/ or to at least one fluid source.

Next to door 7 on the front side 19 there is a display 17 which is configured to provide information about or with reference to the dental or medical device for sanitizing or maintenance (see Fig. 2). The display 17 comprises a screen 17A, in particular a touch screen or LCD screen. The screen 17A is configured to display alphanumeric symbols and control elements for a user to select operation modes and/ or values of operating parameters. Amongst others screen 17A is configured to display a button to start and stop a sanitizing or maintenance process, a current temperature and pressure value and/ or values of other parameters in a chamber 3 of the dental or medical device 1, in particular during a sanitizing or maintenance process, date and time, the progress of a running sanitizing or maintenance process, errors occurring during or after a sanitizing or maintenance process, control elements to select and/ or adjust values of operational parameters, and a plurality of similar data and control elements substantially referring to the performance and/ or operational status of the dental or medical device 1.

In addition screen 17A is configured to display statistics referring to the performance and/ or operation of the dental or medical device 1 and/ or advices for the user. The statistics comprise amongst others the number of sanitizing or maintenance processes performed in a certain period, the number and/ or type of errors occurring, comparison of values of operational parameters of different sanitizing or maintenance processes, etc. The advices comprise amongst others suggestions how to improve the operation of the dental or medical device 1, how to save energy and/ or sanitizing or maintaining medium, need for servicing or refilling of sanitizing or maintaining medium, need for updating the software of the sterilizer 1, etc.

The display 17 further has at least two, according to Fig. 2 three light sources 18 comprising optical semiconductor elements (LEDs). The light sources 18 are arranged at a distance from the screen 17A.

Each of the light sources 18 comprises a plurality of different LEDs so that each light source 18 can emit visible light with different wave lengths/ colors. The light sources 18 are further configured to emit visible light at different emission modes, for example: a continuous light emitting mode; a discontinuous or flashing light emitting mode; a running or chasing light emitting mode in which the at least two light sources light up in sequence. The light sources 18 are configured to commonly indicate the same information, performance or operating status (at a certain moment) by lighting up in the same color and emission mode.

The information indicated by the light sources 18 may either comprise direct information about the performance or operation of the dental or medical device 1, for example, a sanitizing or maintenance process is running or finished, or may be an advise that information or additional information is shown at the screen 17A, for example, data, statistics and/ or advices as described above.

Figures 3, 6, 7 show sectional views of dental or medical devices 1 for sanitizing or maintenance of a load designed as sterilizers. These sterilizers 1 comprise an outer housing 2, a chamber 3 at least partially arranged in the outer housing 2 and configured to accommodate the load to be sanitized or maintained, and at least one supply device 4 for supply of at least one sanitizing or maintaining medium into the chamber 3 to sanitize or maintain the load. The chamber 3 comprises a loading opening 6 to place the load into and take it out of the chamber 3.

Door 7 (see also Fig. 1) covers or closes the loading opening 6. Preferably, it has a locking device 20 to safely lock door 7 during a sanitizing or maintenance process. Door 7 is hinged to a wall 8 surrounding the loading opening 6 of the chamber 3.

A controller 9 (only shown in Fig. 3) is disposed in the interior of outer housing 2 and is configured to control the (entire) operation of the dental or medical device 1 for sanitizing or maintenance, in particular the sanitizing or maintenance process, the display 17 and/ or a light emission device 5. The controller 9 preferably comprises a processor, in particular a micro-processor. In addition, the controller 9 is preferably configured to control a connection, in particular communicative connection, of the dental or medical device 1 to external devices, in particular the exchange of data with the internet, a server, a storing device and/ or a printer.

The dental or medical devices 1 for sanitizing or maintenance comprise at least one, preferably a plurality of sensors 10 (only one shown in Fig. 3) to monitor the operation of the dental or medical devices 1 and/ or to detect a value of an operational parameter, for example, temperature, pressure and/ or humidity. Preferably, the at least one sensor 10 is arranged in or at the chamber 3, in particular mounted on the wall of the chamber 3. The at least one sensor 10 is communicatively coupled with the controller 9 and configured to transmit the detected value to the controller 9.

The controller 9 is configured to receive the detected value, to process it and to control operation of the dental or medical device 1 for sanitizing or maintenance based on the detected value of at least one operating parameter, in particular to control the sanitizing or maintenance process, the display 17 and/ or the light emission device 5.

The dental or medical devices 1 of Figs. 3, 6 and 7 in addition have a light emission device 5 which is configured to emit visible light to indicate the sanitizing or maintenance status of the load accommodated in the chamber 3.

According to Fig. 3 the light emission device 5 is configured to emit visible light into the interior of the chamber 3 to indicate the sanitizing or maintenance status of the load. The light emission device 5 comprises a light emission source 11 which is arranged outside of the chamber 3, adjacent to the chamber 3. The light emission source 11 is arranged on a rear wall of the chamber 3, opposite to loading opening 6.

The light emission source 11 comprises at least one optical semiconductor element (LED) to generate the visible light. The LED is supplied with electric energy through an electric line 13.

The chamber 3 has an opening 12 in its rear wall, so that at least a portion of the visible light generated by the light source 11 can enter the chamber 3 through the opening 12 to indicate the sanitizing or maintenance status of the load.

For example, the light source 11 is configured to emit green and red light. When the sanitizing or maintenance status of the load is in good order, i.e. no error occurred during a sanitizing or maintenance process and/ or the load is sterile, then the light source 11 emits green light. If there is an error which, for example, results in that the load is not sterile the light source 11 emits red light. The light source 11 is controlled by the controller 9 based on the detected values of operational parameters of the sensor 10 as described above to emit the red or green light.

Due to its proximity to chamber 3 the light source 11 is sealed to withstand the harsh conditions during sanitizing or maintenance. Preferably, the light source is hermetically sealed by an enclosure 21.

In addition, the light source 11 and/ or the enclosure 21 are tightly fixed, for example, through welding, to the chamber 3, in particular to the portion of the chamber wall which surrounds opening 12, so that no sanitizing or maintaining medium can leave the chamber 3 and/ or no pollutant can enter the chamber 3. In addition or alternatively, a glass window can be arranged over opening 12 to cover it and to thus seal opening 12.

The arrangement shown in Figure 4 is substantially equal to the embodiment of Fig. 3 except for the position of the light source 11 and the opening 12. The light source 11 and opening 12 are arranged on a lateral wall of the chamber 3, in particular close to the loading opening 6, to emit visible light into the interior of the chamber 3. This advantageously results in a better visibility of the visible light emitted by the light source 11 for the user.

Figure 5 shows another embodiment having a light emission device 5 with a light emission source 11 which is arranged outside of the chamber 3. Here the light emission source 11 is disposed at a distance from the chamber 3 (and thus not shown) and the light emission device 5 in addition comprises an optical light guide 14 which extends through the opening 12 into the interior of the chamber 3 and is configured to direct at least a portion of the visible light generated by the light source 11 into the chamber 3.

The end of the optical light guide 14 enters the chamber 3 through the rear wall of the chamber 3, opposite to loading opening 6. Accordingly, opening 12 is arranged on the rear wall of the chamber 3.

The opening 12 is sealed, for example, as described above, so that no sanitizing or maintaining medium can leave the chamber 3 and/ or no pollutant can enter the chamber 3. In particular, the end of the optical light guide 14 is fused with glass into a metallic sleeve 22 which is welded to the wall of chamber 3 to form a tight and sealed connection.

Figure 6 shows an alternative embodiment with a light emission device 5 which is configured to emit visible light to indicate the sanitizing or maintenance status of the load accommodated in the chamber 3 and which is arranged on an inner side 7A of the door 7. In particular, the light source 11 of the light emission device 5 which generates the visible light is arranged on the inner side 7A of the door 7.

An opening 23 is provided on the inner side 7A of the door 7 through which the visible light is emitted. The opening 23 is sealed so that no sanitizing or maintaining medium can enter from the chamber 3 through the opening 23 and/ or damage the light emission device 5, in particular the light source 11. The seal comprises a glass window fused into the opening 23, for example. Alternatively, the light source 11 is hermetically sealed or encapsulated by an enclosure as described above and extends through and/ or projects opening 23 to seal opening 23.

The dental or medical device 1 for sanitizing or maintenance shown in Fig. 7 comprises a light emission device 5 having a plurality of light sources 11 which generate the visible light to indicate the sanitizing or maintenance status of the load accommodated in the chamber 3. Further, the light sources 11 are arranged in the interior of the chamber 3, in particular on the inside of a lateral wall of the chamber 3. Obviously, the plurality of light sources 11 or a subset of the plurality of light sources 11 can be arranged on another wall of the chamber 3.

The light sources 11 comprise optical semiconductor elements (LEDs). Either each light source 11 is configured to emit only visible light of one color, for example, only red or green with respect to the example described above, or each light source 11 is configured to emit visible light of a plurality of colors, for example, red and green. While with respect to the first alternative the light sources 11 are configured to light up alternatingly to indicate the status of the load, they are configured to light up commonly to indicate the status of the load with respect to the second alternative.

Again, the chamber 3 comprises an opening 12, wherein an electric line 13 for supplying electric energy to the plurality of light sources 11 extends through the opening 12 into the interior of the chamber 3 to the plurality of light sources 11. The opening 12 is arranged on the rear wall of the chamber 3 but can be provided on a different wall of the chamber 3 as well.

The opening 12 is sealed so that no sanitizing or maintaining medium can leave the chamber 3 and/ or no pollutant can enter the chamber 3. For example, a metallic sleeve 22 is provided through which the electric line 13 extends into the chamber 3 and which is welded to the wall of chamber 3. Preferably, the interior of the sleeve 22 is filled with a sealing material, for example, plastic, glass or ceramic.

Fig. 8 shows a tray 15 which is configured to support the load to be sanitized or maintained and which can be placed into a chamber 3 of a dental or medical device 1 for sanitizing or maintenance. The tray 15 comprises at least a portion of a light emission device 5, in particular a light source 11, which generates visible light to indicate the sanitizing or maintenance status of the load supported on the tray 15.

The tray 15 comprises a base 24 with a plurality of openings 25 or slots for the passage of a sanitizing or maintaining medium and a frame 26 surrounding the base 24. The frame 26 in particular has a wall 26A which is arranged at an angel to the base 24. Preferably, at least a portion of the light emission device 5, in particular the light source 11 is disposed on the frame 26.

Fig. 9 shows a rack 16 which is configured to support the load to be sanitized or maintained and which can be placed into a chamber 3 of a dental or medical device 1 for sanitizing or maintenance. The rack 16 comprises at least a portion of a light emission device 5, in particular a light source 11, which generates visible light to indicate the sanitizing or maintenance status of the load supported by the rack 16. In particular the rack 16 is configured to support one or more trays 28 on which the load to be sanitized or maintained can be placed.

The rack 16 comprises a plurality of rods 27, in particular metallic rods, for example steel rods, which are configured to bear the trays 28. Preferably, at least a portion of the light emission device 5, in particular the light source 11, is arranged on at least one of the rods 27.

Figure 10 shows a dental or medical device 1 for sanitizing or maintenance of a load designed as a maintaining or care device. The maintaining or care device 1 comprises an outer housing 2, a (maintenance) chamber 3 configured to accommodate the load to be maintained, a supply device 4 for supply of at least one maintaining or care medium into the chamber 3 and/ or into the load, for example, a handpiece. The maintaining or care device 1 further comprises a loading opening 6, a door (not shown, only one hinge 7B can be seen for pivotally fixing the door to the maintaining or care device 1), a wall 8 surrounding the chamber 3, a controller (not shown) and a sensor 10. All these elements correspond to elements 1 - 4 and 6 - 10 described above with reference to Figs. 1 - 7 and are thus not described again to avoid repetitions. The maintaining or care device 1 preferably comprises at least one container (not shown) which holds at least one maintaining or care medium.

The maintaining or care device 1 in addition has a light emission device 5 which is configured to emit visible light into the interior of the chamber 3 to indicate the sanitizing or maintenance status of the load accommodated in the chamber 3.

The light emission device 5 comprises a light emission source 11 which is arranged outside of the chamber 3, adjacent to the chamber 3. The light emission source 11 is arranged on a side wall of the chamber 3. According to alternative embodiments, the light emission source 11 can be arranged on any other wall of the chamber 3, or inside the chamber 3 or at a distance from the chamber 3, wherein in particular an optical light guide 14 can be provided which is configured to direct at least a portion of the visible light generated by the light source 11 into the chamber 3. Again, since these alternative embodiments are described above with reference to Figs. 3 - 7 they are not described once more to avoid repetitions.

An opening 12 is provided in the wall of the chamber 3 to accommodate the light source 11 and/ or the optical light guide 14 or an electric line 13 to supply electric energy to the light source 11. Also, these different embodiments are described above with reference to Figs. 3 - 7 and are not repeated in detail.

The light emission source 11 comprises at least one optical semiconductor element (LED) to generate the visible light.

The embodiments described or shown, in particular, serve to depict the invention. The characteristics, disclosed in an embodiment, are therefore not limited to that embodiment, but can rather be combined individually or together with one or more characteristics of one of the other embodiments.

## Claims

1. A dental or medical device (1) for sanitizing or maintenance of a load, comprising: an outer housing (2), a chamber (3) at least partially arranged in the outer housing (2) and configured to accommodate the load to be sanitized or maintained, and at least one supply device (4) for supply of at least one sanitizing or maintaining medium into the chamber (3) to sanitize or maintain the load, **characterized by** a light emission device (5) which is configured to emit visible light into the interior of the chamber (3) to indicate the sanitizing or maintenance status of the load accommodated in the chamber (3).

2. A dental or medical device (1) for sanitizing or maintenance of a load, comprising: an outer housing (2), a chamber (3) at least partially arranged in the outer housing (2) and configured to accommodate the load to be sanitized or maintained, wherein the chamber (3) comprises a loading opening (6) to place the load into the chamber (3) and take the load out of the chamber (3), and at least one supply device (4) for supply of at least one sanitizing or maintaining medium into the chamber (3) to sanitize or maintain the load, **characterized by**
a light emission device (5) which is configured to emit visible light to indicate the sanitizing or maintenance status of the load accommodated in the chamber (3) and which is arranged on an inner side (7A) of a door (7) which closes the loading opening (6) of the chamber (3) or on a wall (8) surrounding the loading opening (6) of the chamber (3).

3. The dental or medical device (1) for sanitizing or maintenance according to Claim 1 or 2, **characterized by**
a controller (9) and at least one sensor (10) communicatively coupled with the controller (9), wherein the sensor (10) is configured to detect a value of at least one operating parameter of the dental or medical device (1) for sanitizing or maintenance and to transmit the detected value to the controller (9), wherein the controller (9) is configured to receive the detected value, to process it and to control the light emission device (5) based on the detected value of at least one operating parameter.

4. The dental or medical device (1) for sanitizing or maintenance according to Claim 3, **characterized in that**
the sensor (10) comprises at least one of: a temperature sensor; a humidity sensor; a pressure sensor; a flowmeter; a timekeeper; a level meter; a conductivity meter.

5. The dental or medical device (1) for sanitizing or maintenance according to Claim 3 or 4, **characterized in that**
based on the detected value of at least one operating parameter the controller (9) is configured to at least one of: turn the light emission device (5) on; turn the light emission device (5) off; vary the light intensity of the visible light emitted by the light emission device (5); vary the wavelength of the visible light emitted by the light emission device (5); operate the light emission device (5) in different light emitting modes; operate the light emission device (5) in a continuous light emitting mode; operate the light emission device (5) in a discontinuous or flashing light emitting mode.

6. The dental or medical device (1) for sanitizing or maintenance according to any one of the Claims 1 and 3 - 5, **characterized in that**
the light emission device (5) comprises a light source (11) which generates the visible light, wherein the light source (11) is arranged in the interior of the chamber (3).

7. The dental or medical device (1) for sanitizing or maintenance according to Claim 6, **characterized in that**
the chamber (3) comprises an opening (12), wherein the light source (11) or an electric line (13) for supplying electric energy to the light source (11) extends through the opening (12) into the interior of the chamber (3).

8. The dental or medical device (1) for sanitizing or maintenance according to any one of the Claims 1 and 3 - 5, **characterized in that**
the light emission device (5) comprises a light source (11) which generates the visible light, wherein the light source (11) is arranged outside of the chamber (3).

9. The dental or medical device (1) for sanitizing or maintenance according to Claim 8, **characterized in that**
the chamber (3) comprises an opening (12), wherein at least a portion of the visible light generated by the light source (11) enters the chamber (3) through the opening (12).

10. The dental or medical device (1) for sanitizing or maintenance according to Claim 9, **characterized in that**
the light emission device (5) comprises an optical light guide (14) which reaches to and/ or extends through the opening (12) and is configured to direct at least a portion of the visible light generated by the light source (11) into the chamber (3).

11. The dental or medical device (1) for sanitizing or maintenance according to any one of the preceding Claims 1 - 5, **characterized in that**
the light emission device (5) comprises a light source (11) which generates the visible light and which is arranged in an opening (12) of the chamber (3) to emit at least a portion of the visible light into the chamber (3).

12. The dental or medical device (1) for sanitizing or maintenance according to Claim 2, **characterized in that**
a light source (11) of the light emission device (5) which generates the visible light and/ or an optical light guide (14) of the light emission device (5) which is configured to direct at least a portion of the visible light generated by the light source (11) is arranged on the inner side (7A) of the door (7).

13. The dental or medical device (1) for sanitizing or maintenance according to any one of the Claims 1 or 3 - 5, **characterized by**
a tray (15) or rack (16) which is configured to support the load to be sanitized or maintained and which can be placed into the chamber (3), wherein at least a portion of the light emission device (5) and/ or a light source (11) of the light emission device (5) which generates the visible light is arranged on the tray (15) or rack (16).

14. The dental or medical device (1) for sanitizing or maintenance according to any one of the preceding Claims, **characterized by**
a display (17) providing information about and/ or indicating an operating status of the dental or medical device (1) for sanitizing or maintenance, wherein the display (17) is arranged on the outer housing (2) and comprises at least two light sources (18) which emit visible light with different wave lengths to provide information and / or indicate the operating status.

15. The dental or medical device (1) for sanitizing or maintenance according to Claim 14, **characterized in that**
the information and/ or operating status comprises at least one of: the dental or medical device (1) for sanitizing or maintenance is turned on or off; a sanitizing or maintenance process is running or finished; a sanitizing or maintenance process is stopped before its regular ending; occurrence of an error; need for servicing; need for refill of at least one sanitizing or maintaining medium; a measured value or range of a parameter of the dental or medical device (1) for sanitizing or maintenance; indication that information, statistics and/ or advices for the user is/ are available.

16. A dental or medical sterilizer (1) for sterilization of a load, comprising: an outer housing (2), a chamber (3) at least partially arranged in the outer housing (2) and configured to accommodate the load to be sterilized, and at least one supply device (4) for supply of at least one sterilizing medium into the chamber (3) to sterilize the load, **characterized by**
a display (17) which is arranged on the outer housing (2) and comprises at least two, preferably three, light sources (18) which are configured to emit visible light with different wave lengths to provide information about the dental or medical device (1) for sanitizing or maintenance.
